# EUROPEAN PATENT SPECIFICATION

(11) **EP 3 344 077 B1**
(45) Date of publication and mention of the grant of the patent: **21.08.2024**
(21) Application number: 16766491.1
(22) Date of filing: 26.08.2016
(51) Int. Cl.: A24C 5/01, A24D 1/20, A24F 40/465, A24F 40/20

(54) **ARTICLE FOR USE WITH APPARATUS FOR HEATING SMOKABLE MATERIAL**
ARTIKEL ZUR VERWENDUNG MIT EINER VORRICHTUNG ZUR ERWÄRMUNG VON RAUCHBAREM MATERIAL
ARTICLE DESTINÉ À ÊTRE UTILISÉ AVEC UN APPAREIL POUR CHAUFFER UNE SUBSTANCE À FUMER

(30) Priority: 31.08.2015 US 201514840703
(43) Date of publication of application: 11.07.2018
(60) Divisional application: 20205544.8
(73) Proprietor: Nicoventures Trading Limited, London WC2R 3LA (GB)
(72) Inventor: WILKE, Andrew P., Madison, Wisconsin 53704 (US); KAUFMAN, Duane A, Hollandale, Wisconsin 53544 (US); ROBEY, Raymond J, Madison, Wisconsin 53711 (US)
(74) Representative: Grey, Ian Michael
(86) International application number: PCT/EP2016/070178
(87) International publication number: WO 2017/036951

(56) References cited:
- WO-A1-2015/177045
- WO-A1-95/27411
- CA-A1- 2 923 377
- CN-U- 203 762 288
- GB-A- 2 504 732

## Description

### Technical Field

The present invention relates to articles for use with apparatus for heating smokable material to volatilise at least one component of the smokable material, to methods of manufacturing such articles, and to systems comprising such articles and apparatuses.

### Background

Smoking articles such as cigarettes, cigars and the like burn tobacco during use to create tobacco smoke. Attempts have been made to provide alternatives to these articles by creating products that release compounds without combusting. Examples of such products are so-called "heat not burn" products or tobacco heating devices or products, which release compounds by heating, but not burning, material. The material may be, for example, tobacco or other non-tobacco products, which may or may not contain nicotine.

WO95/27411 discloses inductive heating systems for smoking articles, in which one of the disclosed smoking articles comprises a tobacco flavour medium with a susceptor in thermal proximity thereto.

### Summary

A first aspect of the present invention provides an article for use with apparatus for heating smokable material to volatilise at least one component of the smokable material, the article being as defined in claim 1.

In an exemplary embodiment, the heating material is in contact with the smokable material.

According to the invention, the wrapper comprises heating material that is heatable by penetration with a varying magnetic field, and the cover comprises an adhesive adhering free ends of the wrapper to each other, and the adhesive comprises heating material that is heatable by penetration with a varying magnetic field.

In an exemplary embodiment, the cover comprises a first wrapper that encircles the smokable material and comprises heating material that is heatable by penetration with a varying magnetic field, a second wrapper that encircles the first wrapper, and an adhesive adhering free ends of the second wrapper to each other. In an exemplary embodiment, the first wrapper consists entirely, or substantially entirely, of the heating material. In an exemplary embodiment, the first wrapper comprises a closed circuit of the heating material that encircles the smokable material. In an exemplary embodiment, the second wrapper may be of a non-electrically-conductive material. In an exemplary embodiment, the second wrapper may be free of heating material.

In an exemplary embodiment, the cover comprises a sheet of material and the heating material on the sheet of material. In an exemplary embodiment, a major surface of the sheet of material is only partially covered by the heating material.

In an exemplary embodiment, the cover comprises a sheet of material and a plurality of discrete portions of the heating material in or on the sheet of material. In an exemplary embodiment, the discrete portions are parallel strips. The strips may be linear or non-linear.

In an exemplary embodiment, the cover comprises at least one connector comprising heating material that is heatable by penetration with a varying magnetic field, wherein the connector is connected to the wrapper, wherein the closed circuit comprises the heating material of the wrapper and the heating material of the at least one connector. In an exemplary embodiment, the article is elongate and cylindrical, the wrapper forms a circumferential outer surface of the article, and the, or each, connector is located at a longitudinal end of the article.

In an exemplary embodiment, the heating material comprises electrically-conductive wire.

In an exemplary embodiment, the cover comprises a plurality of closed circuits of heating material that is heatable by penetration with a varying magnetic field. In an exemplary embodiment, the article is elongate and the closed circuits are spaced-apart in a longitudinal direction of the article.

In an exemplary embodiment, the heating material comprises a plurality of relatively closely-spaced portions of the heating material, and a plurality of less-closely-spaced portions of the heating material.

In an exemplary embodiment, a first part of the closed circuit comprises a plurality of relatively closely-spaced portions of the closed circuit, and a second part of the closed circuit comprises a plurality of less-closely-spaced portions of the closed circuit.

In an exemplary embodiment, the heating material comprises one or more materials selected from the group consisting of an electrically-conductive material, a magnetic material, and a non-magnetic material.

In an exemplary embodiment, the heating material comprises a metal or a metal alloy.

In an exemplary embodiment, the heating material comprises one or more materials selected from the group consisting of aluminium, gold, iron, nickel, cobalt, conductive carbon, graphite, plain-carbon steel, stainless steel, ferritic stainless steel, copper, and bronze.

In an exemplary embodiment, the smokable material comprises tobacco and/or one or more humectants.

In an exemplary embodiment, the article comprises a mouthpiece defining a passageway that is in fluid communication with the smokable material.

In an exemplary embodiment, the article is elongate and cylindrical.

In an exemplary embodiment, the article comprises an annular mass of the smokable material, and the article comprises a liner comprising a closed circuit of heating material that is heatable by penetration with a varying magnetic field, wherein the annular mass of the smokable material is located around the liner so that the liner defines an inner surface of the article.

In respective exemplary embodiments, the liner may have any of the above-described features of the cover. In respective exemplary embodiments, the heating material of the liner may have any of the above-described features of the heating material of the cover.

In an exemplary embodiment, the article comprises a temperature detector for detecting a temperature of the article. In some embodiments, the article comprises one or more terminals connected to the temperature detector for making connection with a temperature monitor of the apparatus in use.

A second aspect of the present invention provides a method of manufacturing an article for use with apparatus for heating smokable material to volatilise at least one component of the smokable material, the method being as defined in claim 11.

In an exemplary embodiment, the cover comprises a sheet of material, and the method comprises printing the heating material on at least one major surface of the sheet of material.

According to the invention, the wrapper comprises heating material that is heatable by penetration with a varying magnetic field, and the providing the cover comprises adhering free ends of the wrapper together using a conductive adhesive that comprises heating material that is heatable by penetration with a varying magnetic field.

In an exemplary embodiment, the providing the cover comprises providing the wrapper around the smokable material so that the wrapper defines the outer surface of the article, and connecting at least one connector comprising heating material that is heatable by penetration with a varying magnetic field to the wrapper so as to create a closed circuit of heating material, the closed circuit comprising the heating material of the wrapper and the heating material of the at least one connector.

A third aspect of the present invention provides a system, comprising:
apparatus for heating smokable material to volatilise at least one component of the smokable material; and
an article for use with the apparatus, wherein the article is according to the first aspect of the present invention.

In an exemplary embodiment, the apparatus comprises an interface for cooperating with the article, and a magnetic field generator for generating a varying magnetic field for penetrating the heating material when the article is cooperating with the interface.

In respective exemplary embodiments, the article of the system may have any of the features of the above-described exemplary embodiments of the article of the first aspect of the present invention.

### Brief Description of the Drawings

Embodiments of the invention will now be described, by way of example only, with reference to the accompanying drawings, in which:
Figure 1 shows a schematic perspective view of an example of an article for use with apparatus for heating smokable material to volatilise at least one component of the smokable material;
Figure 2 shows a schematic cross-sectional view of the article of Figure 1;
Figure 3 shows a schematic perspective view of an example of another article for use with apparatus for heating smokable material to volatilise at least one component of the smokable material;
Figure 4 shows a schematic perspective view of an example of an article not according to the invention for use with apparatus for heating smokable material to volatilise at least one component of the smokable material;
Figure 5 shows a schematic perspective view of an example of another article not according to the invention for use with apparatus for heating smokable material to volatilise at least one component of the smokable material;
Figure 6 shows a schematic perspective view of an example of another article not according to the invention for use with apparatus for heating smokable material to volatilise at least one component of the smokable material;
Figure 7 shows a schematic cross-sectional view of the article of Figure 6;
Figure 8 shows a schematic partial cross-sectional view of an example of another article for use with apparatus for heating smokable material to volatilise at least one component of the smokable material; and
Figure 9 is a flow diagram showing an example of a method of manufacturing an article for use with apparatus for heating smokable material to volatilise at least one component of the smokable material.

### Detailed Description

As used herein, the term "smokable material" includes materials that provide volatilised components upon heating, typically in the form of vapour or an aerosol. "Smokable material" may be a non-tobacco-containing material or a tobacco-containing material. "Smokable material" may, for example, include one or more of tobacco per se, tobacco derivatives, expanded tobacco, reconstituted tobacco, tobacco extract, homogenised tobacco or tobacco substitutes. The smokable material can be in the form of ground tobacco, cut rag tobacco, extruded tobacco, liquid, gel, gelled sheet, powder, or agglomerates. "Smokable material" also may include other, non-tobacco, products, which, depending on the product, may or may not contain nicotine. "Smokable material" may comprise one or more humectants, such as glycerol or propylene glycol.

As used herein, the term "heating material" refers to material that is heatable by penetration with a varying magnetic field.

As used herein, the terms "flavour" and "flavourant" refer to materials which, where local regulations permit, may be used to create a desired taste or aroma in a product for adult consumers. They may include extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, gel, powder, or the like.

Induction heating is a process in which an electrically-conductive object is heated by penetrating the object with a varying magnetic field. The process is described by Faraday's law of induction and Ohm's law. An induction heater may comprise an electromagnet and a device for passing a varying electrical current, such as an alternating current, through the electromagnet. When the electromagnet and the object to be heated are suitably relatively positioned so that the resultant varying magnetic field produced by the electromagnet penetrates the object, one or more eddy currents are generated inside the object. The object has a resistance to the flow of electrical currents. Therefore, when such eddy currents are generated in the object, their flow against the electrical resistance of the object causes the object to be heated. This process is called Joule, ohmic, or resistive heating. An object that is capable of being inductively heated is known as a susceptor.

It has been found that, when the susceptor is in the form of a closed circuit, magnetic coupling between the susceptor and the electromagnet in use is enhanced, which results in greater or improved Joule heating.

Magnetic hysteresis heating is a process in which an object made of a magnetic material is heated by penetrating the object with a varying magnetic field. A magnetic material can be considered to comprise many atomic-scale magnets, or magnetic dipoles. When a magnetic field penetrates such material, the magnetic dipoles align with the magnetic field. Therefore, when a varying magnetic field, such as an alternating magnetic field, for example as produced by an electromagnet, penetrates the magnetic material, the orientation of the magnetic dipoles changes with the varying applied magnetic field. Such magnetic dipole reorientation causes heat to be generated in the magnetic material.

When an object is both electrically-conductive and magnetic, penetrating the object with a varying magnetic field can cause both Joule heating and magnetic hysteresis heating in the object. Moreover, the use of magnetic material can strengthen the magnetic field, which can intensify the Joule heating.

In each of the above processes, as heat is generated inside the object itself, rather than by an external heat source by heat conduction, a rapid temperature rise in the object and more uniform heat distribution can be achieved, particularly through selection of suitable object material and geometry, and suitable varying magnetic field magnitude and orientation relative to the object. Moreover, as induction heating and magnetic hysteresis heating do not require a physical connection to be provided between the source of the varying magnetic field and the object, material deposits on the object such as smokable material residue may be less of an issue, design freedom and control over the heating profile may be greater, and cost may be lower.

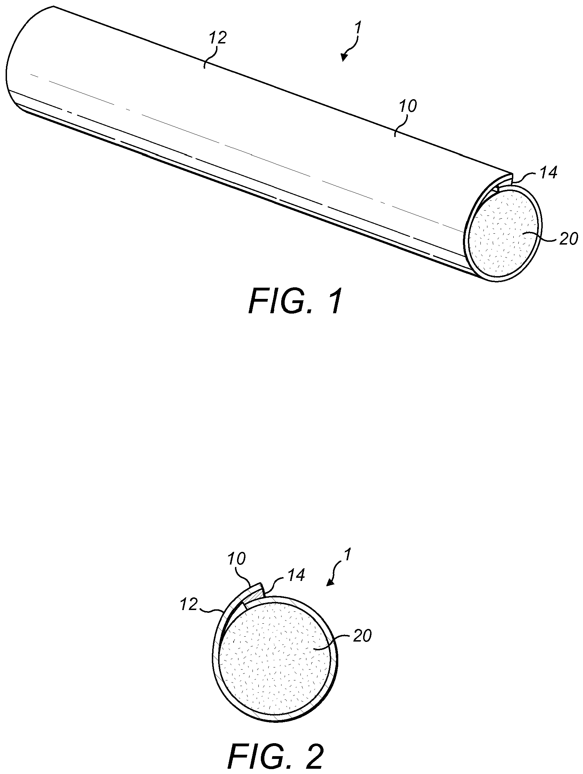

Referring to Figures 1 and 2 there are shown a schematic perspective view and a schematic cross-sectional view of an example of an article according to an embodiment of the invention. The article 1 comprises smokable material 20 and a cover 10 around the smokable material 20. The article 1 is for use with apparatus for heating the smokable material 20 to volatilise at least one component of the smokable material 20 without burning the smokable material 20. An example such apparatus is described below. The cover 10 defines an outer surface of the article 1, which may contact the apparatus in use. Moreover, the cover 10 comprises heating material that is heatable by penetration with a varying magnetic field, as will be described in more detail below. The heating material may be heatable in use to heat the smokable material 20. In this embodiment, the cover 10 comprises a closed circuit of the heating material.

In this embodiment, the article 1 is elongate and cylindrical with a substantially circular cross section. However, in other embodiments, the article may have a cross section other than circular and/or not be elongate and/or not be cylindrical. In this embodiment, the article 1 has proportions approximating those of a cigarette.

In this embodiment, the cover 10 comprises a wrapper 12 comprising the heating material. The wrapper 12 may consist entirely, or substantially entirely, of the heating material. In this embodiment, the wrapper 12 consists of a sheet of foil of the heating material. In this embodiment, the wrapper 12 is wrapped around the smokable material 20 so that free ends of the wrapper 12 overlap each other. The wrapper 12 thus forms all of, or a majority of, a circumferential outer surface of the article 1. The free ends of the wrapper 12 may contact each other, so that the wrapper 12 itself forms the closed circuit of heating material that encircles the smokable material 20.

The cover 10 of this embodiment also comprises an adhesive 14 that adheres the overlapped free ends of the wrapper 12 to each other. The adhesive comprises heating material that is heatable by penetration with a varying magnetic field. The adhesive may comprise one or more of, for example, gum Arabic, natural or synthetic resins, starches, and varnish. The adhesive may comprise one or more powders or fibres of, for example, copper, silver and graphite. The adhesive helps prevent the overlapped free ends of the wrapper 12 from separating, and creates a more robust and reliable closed circuit of heating material that encircles the smokable material 20. In this embodiment, the closed circuit comprises the heating material of the sheet of foil 12 and the heating material of the adhesive 14. In other embodiments, the adhesive 14 may be omitted, or the closed circuit may not comprise the adhesive 14. In such other embodiments, the closed circuit may be formed only by the wrapper 12 itself. The combination of the wrapper 12 and the adhesive 14 may define an outer surface of the article 1, which may contact the apparatus in use. It is to be noted that the size of the adhesive 14 relative to the wrapper 12 is accentuated in Figures 1 and 2 for clarity.

The sheet of foil 12 of this embodiment comprises aluminium. When exposed to air, an aluminium surface oxidises over time. As a result, were either or both of the contacting surfaces of the overlapped free ends of the wrapper 12 to comprise a layer of aluminium oxide, then the closed circuit of heating material around the smokable material 20 could become incomplete or open. In this embodiment, before much, if any, aluminium oxide formed on the surfaces of the overlapped free ends of the sheet of foil 12, the free ends of the sheet of foil 12 were adhered to each other by the electrically-conductive adhesive 14. As noted above, this creates a more robust and reliable closed circuit of heating material.

In other embodiments, the heating material of the wrapper 12 may comprise one or more materials selected from the group consisting of: an electrically-conductive material, a magnetic material, and a non-magnetic material. In some embodiments, the heating material may comprise a metal or a metal alloy. In some embodiments, the heating material may comprise one or more materials selected from the group consisting of: aluminium, gold, iron, nickel, cobalt, conductive carbon, graphite, plain-carbon steel, stainless steel, ferritic stainless steel, copper, and bronze. Other heating material(s) may be used in other embodiments. It has also been found that, when magnetic electrically-conductive material is used as the heating material, magnetic coupling between the magnetic electrically-conductive material and an electromagnet of the apparatus in use may be enhanced. In addition to potentially enabling magnetic hysteresis heating, this can result in greater or improved Joule heating of the heating material, and thus greater or improved heating of the smokable material 20.

In this embodiment, the heating material is in contact with the smokable material 20. Thus, when the heating material of the closed circuit is heated by penetration with a varying magnetic field, heat may be transferred directly from the heating material of the closed circuit to the smokable material 20. In some embodiments, the wrapper 12 may comprise a sheet of material, such as paper, and a layer of heating material on a major surface of the sheet of material. In some such embodiments, the layer of heating material may be in contact with the smokable material 20, so that again heat may be transferred directly from the heating material of the closed circuit to the smokable material 20.

In some embodiments, the cover 10 may comprise a mass of thermal insulation radially outwards of the wrapper 12. The thermal insulation may comprise one or more materials selected from the group consisting of: aerogel, vacuum insulation, wadding, fleece, non-woven material, non-woven fleece, woven material, knitted material, nylon, foam, polystyrene, polyester, polyester filament, polypropylene, a blend of polyester and polypropylene, cellulose acetate, paper or card, and corrugated material such as corrugated paper or card. The thermal insulation may additionally or alternatively comprise an air gap. Such thermal insulation can help prevent heat loss to components of the apparatus, and provide more efficient heating of the smokable material within the cover 10. In some embodiments, the insulation may have a thickness of up to one millimetre, such as up to 0.5 millimetres.

The heating material may have a skin depth, which is an exterior zone within which most of an induced electrical current and/or induced reorientation of magnetic dipoles occurs. By providing that the heating material has a relatively small thickness, a greater proportion of the heating material may be heatable by a given varying magnetic field, as compared to heating material having a depth or thickness that is relatively large as compared to the other dimensions of the heating material. Thus, a more efficient use of material is achieved. In turn, costs are reduced.

In some embodiments, which are respective variations to the embodiment of Figures 1 and 2, the cover 10 may comprise first and second wrappers. The first wrapper encircles the smokable material and comprises heating material that is heatable by penetration with a varying magnetic field. The second wrapper encircles the first wrapper. The cover may further comprise an adhesive that adheres free ends of the second wrapper to each other. Such adhering may have the effect of holding both the wrappers in position relative to the smokable material. A benefit of some such embodiments is that the adhesive that adheres the free ends of the second wrapper to each other need not comprise heating material. The adhesive may comprise one or more of, for example, gum Arabic, natural or synthetic resins, starches, and varnish.

The first wrapper may, for example, be the same as the wrapper 12 of the embodiment of Figures 1 and 2, or any of the above-described variations to the wrapper 12 of that embodiment. In some embodiments, the first wrapper consists entirely, or substantially entirely, of the heating material. In some embodiments, the first wrapper may comprise a closed circuit of the heating material that encircles the smokable material. The first wrapper may comprise electrically-conductive material, such as a layer of electrically-conductive material that encircles the smokable material. The heating material of the first wrapper may comprise any one or more of the materials discussed above for the heating material of the wrapper 12.

The second wrapper may be of a non-electrically-conductive material. The second wrapper may be free of heating material. The second wrapper may, for example, be made of paper.

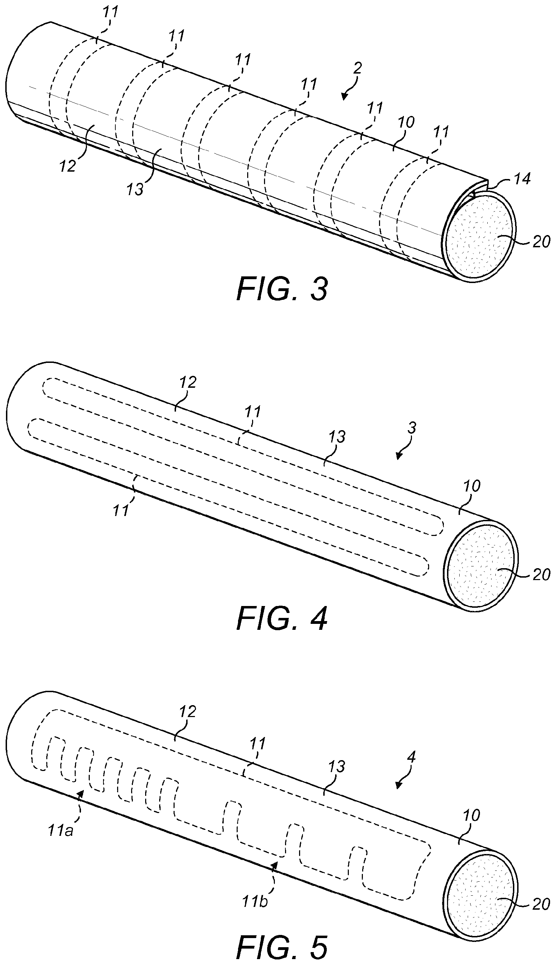

Referring to Figure 3 there is shown a schematic perspective view of an example of another article according to an embodiment of the invention. The article 2 of this embodiment is identical to the article 1 of Figures 1 and 2 except for the form of the cover 10. Any of the above-described possible variations to the article 1 of Figures 1 and 2 may be made to the article 2 of Figure 3 to form separate respective embodiments.

In this embodiment, the wrapper 12 comprises a sheet of material 13 and wires or tracks 11 of heating material on a major surface of the sheet of material 13. Therefore, the major surface of the sheet of material 13 is only partially covered by the heating material. In this embodiment, the wires or tracks 11 are between the sheet of material 13 and the smokable material 20. In this embodiment, the sheet of material 13 comprises a sheet of paper, but in other embodiments the sheet of material 13 may be made of an electrically-insulating material other than paper. In this embodiment, the wrapper 12 is wrapped around the smokable material 20 so that free ends of the wrapper 12 overlap each other. The wrapper 12 thus forms all of, or a majority of, a circumferential outer surface of the article 2.

The cover 10 of this embodiment also comprises an adhesive 14 that adheres the overlapped free ends of the wrapper 12 to each other. The adhesive of this embodiment comprises heating material. The adhesive may be the same as any one of the adhesives discussed above. The adhesive helps prevent the overlapped free ends of the wrapper 12 from separating. The adhesive 14 also connects respective free ends of each of the wires or tracks 11, so as to create a plurality of closed circuits of heatable material that each encircle the smokable material 20. To achieve this, the adhesive may extend around at least one of the free ends of the wrapper 12, or may extend through one or more through holes, or vias, in the wrapper 12. The closed circuits of heating material may be heatable in use to heat the smokable material 20. The combination of the wrapper 12 and the adhesive 14 may define an outer surface of the article 1, which may contact the apparatus in use. It is to be noted that the size of the adhesive 14 relative to the wrapper 12 is accentuated in Figure 3 for clarity.

In this embodiment, the closed circuits are spaced-apart in a longitudinal direction of the article 2. In this embodiment, each of the closed circuits comprises the heating material of one of the wires or tracks 11 and the heating material of the adhesive 14. In other embodiments, the adhesive 14 may be omitted, or the closed circuits may not comprise the electrically-conductive adhesive 14. In such other embodiments, the closed circuits may be formed only by the wires or tracks 11.

The wires or tracks 11 of this embodiment comprise aluminium. In other embodiments, the electrically-conductive material of the wires or tracks 11 may comprise any one or more of the materials discussed above for the heating material of earlier embodiments.

In this embodiment, the wires or tracks 11 are in contact with the smokable material 20. Thus, when the electrically-conductive material of the closed circuits is heated by induction heating, heat may be transferred directly from the electrically-conductive material of the closed circuits to the smokable material 20. However, in other embodiments, the wires or tracks 11 may be kept out of contact with the smokable material 20. For example, the sheet of material 13 may be located between the wires or tracks 11 and the smokable material 20. In some embodiments, the wires or tracks 11 may be embedded within the sheet of material 13.

In this embodiment, the wires or tracks 11 are of substantially equal axial width. In other embodiments, the tracks 11 may be of different respective widths. Regions of the smokable material 20 adjacent the wider tracks may then be heated to a greater degree by the application of a given varying magnetic field than regions of the smokable material 20 adjacent the narrower tracks.

Referring to Figure 4 there is shown a schematic perspective view of an example of an article not according to the invention. The article 3 of this embodiment is identical to the article 1 of Figures 1 and 2 except for the form of the cover 10. Any of the above-described possible variations to the article 1 of Figures 1 and 2 may be made to the article 3 of Figure 4 to form separate respective embodiments.

In this embodiment, the cover 10 comprises a wrapper 12 comprising heating material. More specifically, the wrapper 12 comprises a sheet of material 13 and wires or tracks 11 of heating material on a major surface of the sheet of material 13. Therefore, the major surface of the sheet of material 13 is only partially covered by the heating material. In this embodiment, the wires or tracks 11 are between the sheet of material 13 and the smokable material 20. In this embodiment, the sheet of material 13 comprises a sheet of paper, but in other embodiments the sheet of material 13 may be made of an electrically-insulating material other than paper. In this embodiment, the wrapper 12 is wrapped around the smokable material 20 so that free ends of the wrapper 12 overlap each other. The wrapper 12 thus forms all of, or a majority of, a circumferential outer surface of the article 3.

The cover 10 of this embodiment may also comprise an adhesive (not shown) that adheres the overlapped free ends of the wrapper 12 to each other to help prevent them from separating. In other embodiments, the adhesive may be omitted. When such adhesive is present, the combination of the wrapper 12 and the adhesive may define an outer surface of the article 1, which may contact the apparatus in use.

In this embodiment, the wires or tracks 11 define a plurality of closed circuits of the heating material. That is, the wrapper 12 comprises a plurality of closed circuits of the heating material, each of which closed circuits is formed by one of the wires or tracks 11. The closed circuits of heating material may be heatable in use to heat the smokable material 20. In this embodiment, the closed circuits are elongate in a direction parallel to the longitudinal dimension of the article 3, and are spaced-apart in a circumferential direction of the article 3. In other embodiments, the wires or tracks 11, and thus the closed circuits, may be arranged differently relative to each other or to the sheet of material 13.

The wires or tracks 11 of this embodiment comprises aluminium. In other embodiments, the heating material of the wires or tracks 11 may comprise any one or more of the materials discussed above for the heating material of earlier embodiments.

In this embodiment, the wires or tracks 11 are in contact with the smokable material 20. Thus, when the heating material of the closed circuits is heated by penetration with a varying magnetic field, heat may be transferred directly from the heating material of the closed circuits to the smokable material 20. However, in other embodiments, the wires or tracks 11 may be kept out of contact with the smokable material 20, as discussed above.

Referring to Figure 5 there is shown a schematic perspective view of an example of another article not according to the invention. The article 4 of this embodiment is identical to the article 3 of Figure 4 except for the form of the cover 10. Any of the above-described possible variations to the article 3 of Figure 4 may be made to the article 4 of Figure 5 to form separate respective embodiments.

In this embodiment, the cover 10 comprises a wrapper 12 comprising a sheet of material 13 and a wire or track 11 of heating material on a major surface of the sheet of material 13. Therefore, the major surface of the sheet of material 13 is only partially covered by the heating material. In this embodiment, the wire or track 11 is between the sheet of material 13 and the smokable material 20. In this embodiment, the wrapper comprises only a single wire or track 11 of heating material on the sheet of material 13. However, in other embodiments, the wrapper 12 may comprise a plurality of wires or tracks 11 of heating material on the sheet of material 13.

In this embodiment, the wire or track 11 defines a closed circuit of the heating material. The closed circuit of heating material may be heatable in use to heat the smokable material 20. In this embodiment, overall, the closed circuit is elongate in a direction parallel to the longitudinal dimension of the article 3. However, the wire or track 11 follows a tortuous path on the sheet of material 13. More particularly, in this embodiment, a first part 11a of the closed circuit comprises a plurality of relatively closely-spaced portions of the closed circuit, and a second part 11b of the closed circuit comprises a plurality of less-closely-spaced portions of the circuit. Thus, the heating material comprises a plurality of relatively closely-spaced portions, and a plurality of less-closely-spaced portions. Accordingly, when heated by penetration with a varying magnetic field, the wire or track 11 emits more heat per unit area at the first part 11a of the closed circuit than at the second part 11b of the closed circuit. Thus, progressive heating of the smokable material 20, and thereby progressive generation of vapour, is achievable.

More specifically, the first part 11a of the closed circuit is able to heat a first region of the smokable material 20 relatively quickly to initialise volatilisation of at least one component of the smokable material 20 and formation of vapour in the first region of the smokable material 20. The second part 11b of the closed circuit is able to heat a second region of the smokable material 20 relatively slowly to initialise volatilisation of at least one component of the smokable material 20 and formation of vapour in the second region of the smokable material 20. Accordingly, vapour is able to be formed relatively rapidly for inhalation by a user, and vapour can continue to be formed thereafter for subsequent inhalation by the user even after the first region of the smokable material 20 may have ceased generating vapour. The first region of the smokable material 20 may cease generating the vapour when it becomes exhausted of volatilisable components of the smokable material 20.

The wire or track 11 of this embodiment comprises aluminium. In other embodiments, the electrically-conductive material of the wire or track 11 may comprise any one or more of the materials discussed above for the heating material of earlier embodiments.

In this embodiment, the wire or track 11 are in contact with the smokable material 20. Thus, when the heating material of the closed circuit is heated by penetration with a varying magnetic field, heat may be transferred directly from the heating material of the closed circuit to the smokable material 20. However, in other embodiments, the wire or track 11 may be kept out of contact with the smokable material 20, as discussed above.

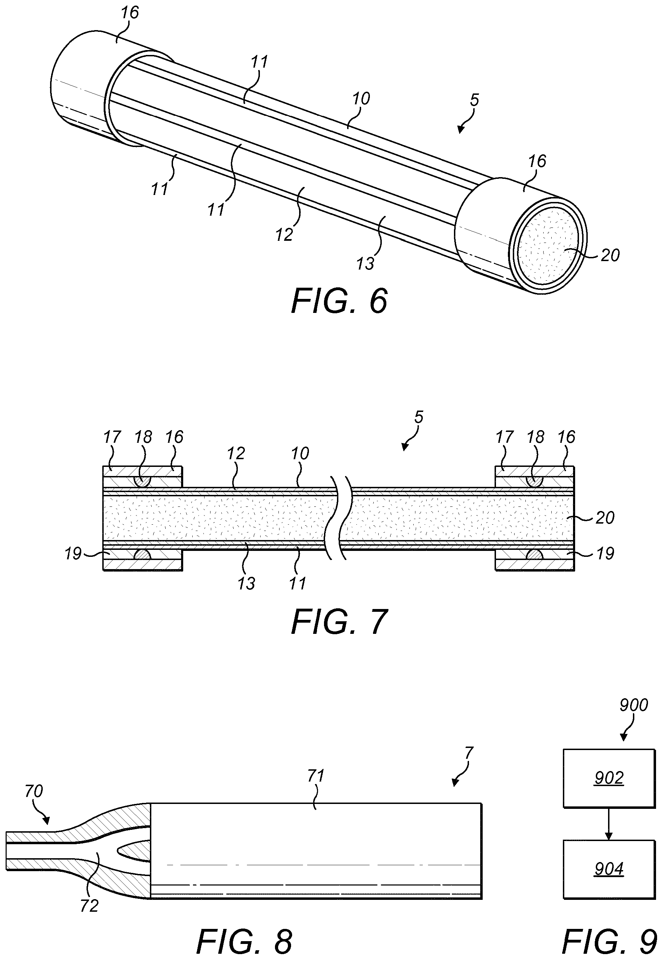

Referring to Figures 6 and 7 there are shown a schematic perspective view and a schematic cross-sectional view of an example of another article not according to the invention. The article 5 comprises smokable material 20 and a cover 10 around the smokable material 20. The article 5 is for use with apparatus for heating the smokable material 20 to volatilise at least one component of the smokable material 20 without burning the smokable material 20. The cover 10 defines an outer surface of the article 5, which may contact the apparatus in use. Moreover, the cover 10 comprises a plurality of closed circuits of heating material, as will be described in more detail below.

In this embodiment, the article 5 is elongate and cylindrical with a substantially circular cross section. However, in other embodiments, the article may have a cross section other than circular and/or not be elongate and/or not be cylindrical. In this embodiment, the article 5 has proportions approximating those of a cigarette.

In this embodiment, the cover 10 comprises a wrapper 12 comprising heating material. More specifically, the wrapper 12 comprises a sheet of material 13 and discrete wires or tracks 11 of heating material on a major surface of the sheet of material 13. Therefore, the major surface of the sheet of material 13 is only partially covered by the heating material. In this embodiment, the sheet of material 13 is between the wires or tracks 11 and the smokable material 20. In this embodiment, each of the wires or tracks 11 is linear and parallel to the longitudinal dimension of the article 5, and the wires or tracks 11 are spaced-apart in a circumferential direction of the article 5. However, in other embodiments, the wires or tracks 11 may be non-linear and/or nonparallel to the longitudinal dimension of the article 5.

In this embodiment, the sheet of material 13 comprises a sheet of paper, but in other embodiments the sheet of material 13 may be made of an electrically-insulating material other than paper. In this embodiment, the wrapper 12 is wrapped around the smokable material 20 so that free ends of the wrapper 12 overlap each other. The wrapper 12 thus forms part of a circumferential outer surface of the article 5.

The cover 10 of this embodiment may also comprise an adhesive (not shown) that adheres the overlapped free ends of the wrapper 12 to each other to help prevent them from separating. In other embodiments, the adhesive may be omitted.

In this embodiment, the cover 10 also comprises two connectors 16, each of which is located at a longitudinal end of the article 5. However, in other embodiments, one or each of the connectors 16 may be located elsewhere along the length of the article 5. In this embodiment, each of the connectors 16 comprises a strip of material 17 and one or more bodies 18 of heating material on the strip of material 17. In this embodiment, the strip of material 17 is a strip of paper, but in other embodiments the strip of material 17 may be made of an electrically-insulating material other than paper. In this embodiment, the connectors 16 are adhered to the wrapper 12 by an adhesive 19, which may be an adhesive comprising heating material. In this embodiment, the combination of the wrapper 12 and the connectors 16 defines an outer surface of the article 5, which may contact the apparatus in use. It is to be noted that the size of the adhesive 19, connectors 16 and components thereof relative to the wrapper 12 is accentuated in Figure 7 for clarity.

In this embodiment, the connectors 16 are arranged so that at least two of the wires or tracks 11 on the sheet of material 13 are connected by the bodies 18 of heating material of both the connectors 16, so as to form a plurality of closed circuits of heating material. In a variation to this embodiment, only two wires or tracks 11 may be provided on the sheet of material 13, and those two wires or tracks 11 may be connected by the bodies 18 of heating material of both the connectors 16, so as to form just one closed circuit of heating material. The, or each, closed circuit of heating material may be heatable in use to heat the smokable material 20.

The wires or tracks 11 of this embodiment comprise aluminium. In other embodiments, the electrically-conductive material of the wires or tracks 11 may comprise any one or more of the materials discussed above for the heating material of earlier embodiments.

In this embodiment, the wires or tracks 11 are in contact with the smokable material 20. Thus, when the heating material of the closed circuits is heated by penetration with a varying magnetic field, heat may be transferred directly from the heating material of the closed circuits to the smokable material 20. However, in other embodiments, the wires or tracks 11 may be kept out of contact with the smokable material 20. For example, the sheet of material 13 may be located between the wires or tracks 11 and the smokable material 20. In some embodiments, the wires or tracks 11 may be embedded within the sheet of material 13.

In a variation to the illustrated embodiment, the wires or tracks 11 may each be U-shaped, with the free ends of the arms of the U located at one longitudinal end of the article 5. In such a variation, only one of the connectors 16 need be provided at that one longitudinal end of the article 5 to create one or more closed circuits comprising the heating material of the wires or tracks 11 and the body 18 of the connector 16.

In a variation to the illustrated embodiment, the connector(s) 16 may take a different form to that shown in Figures 6 and 7. For example, in some embodiments, the, or each, connector may abut a longitudinal end of the combination of the smokable material 20 and wrapper 12, so as to form a longitudinal end of the article 5. In such a variation, the, or each, connector 16 may comprise heating material that abuts the associated longitudinal end of the combination of the smokable material 20 and wrapper 12 and connects at least two of the wires or tracks 11 on the sheet 13.

In some embodiments, the connector(s) may be movable or rotatable relative to the wires or tracks 11 on the sheet 13. Such rotation may enable the number of connections between the wires or tracks 11 made by the connector(s) to be changed, thereby to vary the amount of heating material present in a circuit or circuits, which may vary the intensity of heating provided to the smokable material 20. In some embodiments, the tracks 11 may be of different thicknesses and/or widths, so that different heating profiles may be created by rotating the connector(s) to different rotational positions relative to the tracks 11.

In respective variations to this embodiment, the connector(s) may be omitted. In such embodiments, the article may comprise the smokable material 20 and the cover 10 around the smokable material 20, wherein the cover 10 defines an outer surface of the article. The cover may comprise a sheet of material and a plurality of discrete portions of heating material that is heatable by penetration with a varying magnetic field. The discrete portions may be in or on the sheet of material. The discrete portions may be between the sheet and the smokable material, or the sheet may be between the discrete portions and the smokable material.

The discrete portions may be parallel strips or other than parallel strips, such as discrete patches of the heating material. Such parallel strips may be linear or non-linear. The discrete nature of the discrete portions provides thermal breaks between the discrete portions, to control the degree to which different regions of the smokable material 20 are heated in use. This may help progressive heating of the smokable material 20, and thus progressive generation of vapour, to be achieved. The heating material may comprise a plurality of relatively closely-spaced discrete portions, and a plurality of less-closely-spaced discrete portions. Accordingly, when heated by penetration with a varying magnetic field, the closely-spaced discrete portions emit more heat per unit area than the less-closely-spaced discrete portions. Thus, progressive heating of the smokable material 20, and thereby progressive generation of vapour, is achievable.

In some embodiments, the article comprises an annular mass of the smokable material 20, and the article comprises a liner comprising a closed circuit of heating material, wherein the annular mass of the smokable material 20 is located around the liner so that the liner defines an inner surface of the article. The liner may have any of the above-described features of the cover. The heating material of the liner may have any of the above-described features of the heating material of the cover. The closed circuit of heating material of the liner may be heatable in use, to further or more effectively heat the smokable material 20 of the article.

In some embodiments, the heating material may not be susceptible to eddy currents being induced therein by penetration with a varying magnetic field. In such embodiments, the heating material may be a magnetic material that is non-electrically-conductive, and thus may be heatable by the magnetic hysteresis process discussed above.

In some embodiments, the article comprises a mouthpiece defining a passageway that is in fluid communication with the smokable material 10. Referring to Figure 8, there is shown a schematic partial cross-sectional view of an example of an article 7 according to an embodiment of the invention. The section of the article 7 numbered 71 could comprise any of the constructions shown in Figures 1 to 7 or any of the variants thereof discussed above. The mouthpiece 70 and passageway 72 thereof are shown connected to the construction with the passageway 72 aligned so as to be in fluid communication with the smokable material 10 of the construction. The mouthpiece 70 may be made of any suitable material, such as a plastics material, cardboard, or rubber.

In use, when the smokable material 10 is heated by the heated heating material of the article, volatilised components of the smokable material 10 can be readily inhaled by a user. In embodiments in which the article is a consumable article, once all or substantially all of the volatilisable component(s) of the smokable material 10 in the article has/have been spent, the user may dispose of the mouthpiece together with the rest of the article. This can be more hygienic than using the same mouthpiece with multiple articles, can help ensure that the mouthpiece is correctly aligned with the smokable material, and presents a user with a clean, fresh mouthpiece each time they wish to use another article.

The mouthpiece 70, when provided, may comprise or be impregnated with a flavourant. The flavourant may be arranged so as to be picked up by heated vapour as the vapour passes through the passageway 72 of the mouthpiece 70 in use.

Referring to Figure 9 there is shown a flow diagram of an example of a method according to an embodiment of the invention of manufacturing an article for use with apparatus for heating smokable material to volatilise at least one component of the smokable material. The method may be used to manufacture one of the above-described articles 1, 2, 3, 4, 5.

The method 900 comprises providing 902 the smokable material 20, and providing 904 the cover 10 around the smokable material 20 so that the cover 10 defines the outer surface of the article 1, 2, 3, 4, 5.

In some embodiments in which the cover 10 comprises a sheet of material 13, the method may comprise printing heating material on at least one major surface of the sheet of material 13. Such printing may be used to provide the wires or tracks 11 on the sheet of material 13 of the articles 2, 3, 4, 5 shown in Figures 3 to 7, for example.

In some embodiments, the providing 904 the cover 10 around the smokable material 20 may comprise providing a wrapper 12 comprising heating material around the smokable material 20, and may comprise adhering free ends of the wrapper 12 together using an adhesive 14 comprising heating material. Such a process may be used in the manufacture of the articles 1, 2 shown in Figures 1 to 3, for example. The process may be based on a known process for rolling cigarettes.

In some embodiments, the providing 904 the cover 10 may comprise providing a wrapper 12 comprising heating material around the smokable material 20 so that the wrapper 12 defines an outer surface of the article 5. The providing 904 the cover 10 may further comprise connecting at least one connector 16 comprising heating material 18 to the wrapper 12 so as to create a closed circuit of heating material, the closed circuit comprising the heating material of the wrapper 12 and the heating material of the at least one connector 16. Such a process may be used in the manufacture of the article 5 shown in Figures 6 and 7, for example.

Each of the above-described articles 1, 2, 3, 4, 5 and described variants thereof may be used with an apparatus for heating the smokable material 20 to volatilise at least one component of the smokable material 20. The apparatus may be to heat the smokable material 20 to volatilise the at least one component of the smokable material 20 without burning the smokable material 20. Any one of the article(s) 1, 2, 3, 4, 5 and such apparatus may be provided together as a system. The system may take the form of a kit, in which the article 1, 2, 3, 4, 5 is separate from the apparatus. Alternatively, the system may take the form of an assembly, in which the article 1, 2, 3, 4, 5 is combined with the apparatus.

The apparatus may comprise a magnetic field generator for generating a varying magnetic field for heating the heating material of the article 1, 2, 3, 4, 5, such as the foil 12 of the article 1 shown in Figures 1 and 2, or the wire(s) or track(s) of the articles 2, 3, 4, 5 shown in Figures 3 to 7. Such a magnetic field generator may comprise an electrical power source, an electromagnet, a device for passing a varying electrical current, such as an alternating current, through the coil, a controller, and a user interface for user-operation of the controller. The electrical power source may be a rechargeable battery, a non-rechargeable battery, a connection to a mains electricity supply, or the like.

The coil may take any suitable form, such as a helical coil of electrically-conductive material, such as copper. The magnetic field generator may comprise a magnetically permeable core around which the coil is wound, to concentrate the magnetic flux produced by the coil and make a more powerful magnetic field. The magnetically permeable core may be made of iron, for example. In some embodiments, the magnetically permeable core may extend only partially along the length of the coil, so as to concentrate the magnetic flux only in certain regions.

The device for passing a varying electrical current through the coil may be electrically connected between the electrical power source and the coil. The controller may be electrically connected to the electrical power source, and be communicatively connected to the device to control the device, so as to control the supply of electrical power from the electrical power source to the coil. In some embodiments, the controller may comprise an integrated circuit (IC), such as an IC on a printed circuit board (PCB). In other embodiments, the controller may take a different form. In some embodiments, the apparatus may have a single electrical or electronic component comprising the device and the controller. The controller may be operated by user-operation of the user interface, which may comprise a push-button, a toggle switch, a dial, a touchscreen, or the like. Operation of the user interface by a user may cause the controller to cause the device to apply an alternating electric current across the coil, so as to cause the coil to generate an alternating magnetic field.

The apparatus may have a recess or other interface for receiving the article 1, 2, 3, 4, 5, and the coil may be positioned relative to the recess or interface so that the varying or alternating magnetic field produced by the coil in use penetrates the recess or interface at a location corresponding to the heating material of the article 1, 2, 3, 4, 5 when the article 1, 2, 3, 4, 5 is in the recess or cooperating with the interface. When the heating material of the article 1, 2, 3, 4, 5 is an electrically-conductive material, this may cause the generation of one or more eddy currents in the heating material of the article 1, 2, 3, 4, 5. The flow of eddy currents against the electrical resistance of the heating material of the article 1, 2, 3, 4, 5 causes the heating material of the article 1, 2, 3, 4, 5 to be heated by Joule heating. When the heating material of the article 1, 2, 3, 4, 5 is a magnetic material, the orientation of magnetic dipoles in the heating material changes with the changing applied magnetic field, which causes heat to be generated in the heating material of the article 1, 2, 3, 4, 5 by magnetic hysteresis heating.

The apparatus may have a mechanism for compressing the article 1, 2, 3, 4, 5 when the article 1, 2, 3, 4, 5 is inserted in the recess or cooperating with the interface. Such compression of the article 1, 2, 3, 4, 5 can compress the smokable material 20, so as to increase the thermal conductivity of the smokable material 20. In other words, compression of the smokable material 20 can provide for higher heat transfer through the article 1, 2, 3, 4, 5.

The apparatus may have a temperature sensor for sensing a temperature of the recess, interface, or article 1, 2, 3, 4, 5 in use. The temperature sensor may be communicatively connected to the controller, so that the controller is able to monitor the temperature. In some embodiments, the temperature sensor may be arranged to take an optical temperature measurement of the recess, interface or article. In some embodiments, the article 1, 2, 3, 4, 5 may comprise a temperature detector, such as a resistance temperature detector (RTD), for detecting a temperature of the article 1, 2, 3, 4, 5. The article 1, 2, 3, 4, 5 may further comprise one or more terminals connected, such as electrically-connected, to the temperature detector. The terminal(s) may be for making connection, such as electrical connection, with a temperature monitor of the apparatus when the article 1, 2, 3, 4, 5 is in the recess or cooperating with the interface. The controller may comprise the temperature monitor. The temperature monitor of the apparatus may thus be able to determine a temperature of the article 1, 2, 3, 4, 5 during use of the article 1, 2, 3, 4, 5 with the apparatus.

In some embodiments, by providing that the heating material of the article 1, 2, 3, 4, 5 has a suitable resistance, the response of the heating material to a change in temperature could be sufficient to give information regarding temperature inside the article 1, 2, 3, 4, 5. The temperature sensor of the apparatus may then comprise a probe for analysing the heating material.

On the basis of one or more signals received from the temperature sensor or temperature detector, the controller may cause the device to adjust a characteristic of the varying or alternating current passed through the coil as necessary, in order to ensure that the temperature remains within a predetermined temperature range. The characteristic may be, for example, amplitude or frequency. Within the predetermined temperature range, in use the smokable material 20 within an article 1, 2, 3, 4, 5 inserted in the recess or cooperating with the interface may be heated sufficiently to volatilise at least one component of the smokable material 20 without combusting the smokable material 20. In some embodiments, the temperature range is about 50°C to about 250°C, such as between about 50°C and about 150°C, between about 50°C and about 120°C, between about 50°C and about 100°C, between about 50°C and about 80°C, or between about 60°C and about 70°C. In some embodiments, the temperature range is between about 170°C and about 220°C. In other embodiments, the temperature range may be other than this range. The apparatus may have a delivery device for delivering the volatilised component(s) of the smokable material 20 to a user.

The apparatus may define an air inlet that fluidly connects the recess or interface with an exterior of the apparatus. A user may be able to inhale the volatilised component(s) of the smokable material by drawing the volatilised component(s) through a channel, such as a channel of a mouthpiece of the apparatus. As the volatilised component(s) are removed from the article 1, 2, 3, 4, 5, air may be drawn into the recess or interface via the air inlet of the apparatus.

The apparatus may provide haptic feedback to a user. The feedback could indicate that heating of the susceptor is taking place, or be triggered by a timer to indicate that greater than a predetermined proportion of the original quantity of volatilisable component(s) of the smokable material 20 in the article 1, 2, 3, 4, 5 has/have been spent, or the like. The haptic feedback could be created by interaction of the susceptor with the coil (i.e. magnetic response), by interaction of an electrically-conductive element with the coil, by rotating an unbalanced motor, by repeatedly applying and removing a current across a piezoelectric element, or the like.

The apparatus may comprise more than one coil. The plurality of coils could be operated to provide progressive heating of the smokable material 20 in an article 1, 2, 3, 4, 5, and thereby progressive generation of vapour. For example, one coil may be able to heat a first region of the heating material relatively quickly to initialise volatilisation of at least one component of the smokable material 20 and formation of vapour in a first region of the smokable material 20. Another coil may be able to heat a second region of the heating material relatively slowly to initialise volatilisation of at least one component of the smokable material 20 and formation of vapour in a second region of the smokable material 20. Accordingly, vapour is able to be formed relatively rapidly for inhalation by a user, and vapour can continue to be formed thereafter for subsequent inhalation by the user even after the first region of the smokable material 20 may have ceased generating vapour. The initially-unheated second region of smokable material 20 could act as a filter, to reduce the temperature of created vapour or make the created vapour mild, during heating of the first region of smokable material 20.

In some embodiments, the heating material of the cover may comprise discontinuities or holes therein. Such discontinuities or holes may act as thermal breaks to control the degree to which different regions of the smokable material are heated in use. Areas of the heating material with discontinuities or holes therein may be heated to a lesser extent that areas without discontinuities or holes. This may help progressive heating of the smokable material, and thus progressive generation of vapour, to be achieved.

In each of the above described embodiments, the smokable material 20 comprises tobacco. However, in respective variations to each of these embodiments, the smokable material 20 may consist of tobacco, may consist substantially entirely of tobacco, may comprise tobacco and smokable material other than tobacco, may comprise smokable material other than tobacco, or may be free of tobacco. In some embodiments, the smokable material 20 may comprise a vapour or aerosol forming agent or a humectant, such as glycerol, propylene glycol, triactein, or diethylene glycol.

An article embodying the present invention may be a cartridge or a capsule, for example.

In each of the above described embodiments, the article 1, 2, 3, 4, 5 is a consumable article. Once all, or substantially all, of the volatilisable component(s) of the smokable material 20 in the article 1, 2, 3, 4, 5 has/have been spent, the user may remove the article 1, 2, 3, 4, 5 from the apparatus and dispose of the article 1, 2, 3, 4, 5. The user may subsequently re-use the apparatus with another of the articles 1, 2, 3, 4, 5. However, in other respective embodiments, the article 1, 2, 3, 4, 5 may be nonconsumable, and the apparatus and the article 1, 2, 3, 4, 5 may be disposed of together once the volatilisable component(s) of the smokable material 20 has/have been spent.

In some embodiments, the apparatus discussed above is sold, supplied or otherwise provided separately from the articles 1, 2, 3, 4, 5 with which the apparatus is usable. However, in some embodiments, the apparatus and one or more of the articles 1, 2, 3, 4, 5 may be provided together as a system, such as a kit or an assembly, possibly with additional components, such as cleaning utensils.

The invention could be implemented in a system comprising any one of the articles discussed herein, and any one of the apparatuses discussed herein, wherein the apparatus itself further has heating material, such as in a susceptor, for heating by penetration with the varying magnetic field generated by the magnetic field generator. Heat generated in the heating material of the apparatus itself could be transferred to the article to further heat the smokable material therein.

In order to address various issues and advance the art, the entirety of this disclosure shows by way of illustration and example various embodiments in which the claimed invention may be practised and which provide for superior articles for use with apparatus for heating smokable material to volatilise at least one component of the smokable material, superior methods of manufacturing such articles, and superior systems comprising such articles and such apparatus. The advantages and features of the disclosure are of a representative sample of embodiments only, and are not exhaustive and/or exclusive. They are presented only to assist in understanding and teach the invention as defined in the claims.

## Claims

1. An article for use with an apparatus for heating smokable material to volatilize at least one component of the smokable material, the article comprising:
smokable material (20); and
a cover (10) around the smokable material;
wherein the cover defines an outer surface of the article and comprises a wrapper (12), wherein the cover comprises a closed circuit of heating material that is heatable by penetration with a varying magnetic field, wherein the closed circuit is disposed such that the closed circuit encircles the smokable material, and wherein the cover comprises an adhesive (14) adhering free ends of the wrapper to each other, and **characterized in that** the adhesive comprises heating material that is heatable by penetration with a varying magnetic field.

2. The article of claim 1, wherein the heating material is in contact with the smokable material.

3. The article of claim 1, wherein the cover comprises:
a first wrapper that encircles the smokable material, the first wrapper comprising heating material that is heatable by penetration with a varying magnetic field, a second wrapper that encircles the first wrapper, and an adhesive adhering free ends of the second wrapper to each other.

4. The article of claim 1, wherein the cover comprises a sheet of material (13), wherein the heating material is disposed on the sheet of material;
optionally wherein a major surface of the sheet of material is only partially covered by the heating material.

5. The article of claim 1, wherein the cover comprises a sheet of material (13) and a plurality of discrete portions of the heating material in or on the sheet of material;
optionally wherein the discrete portions are parallel strips.

6. The article of claim 1, wherein the wrapper comprises heating material that is heatable by penetration with a varying magnetic field, and
wherein the cover comprises at least one connector (16) comprising heating material that is heatable by penetration with a varying magnetic field, wherein the connector is connected to the wrapper, wherein the closed circuit comprises the heating material of the wrapper and the heating material of the at least one connector;
optionally wherein the article is elongate and cylindrical, the wrapper forms a circumferential outer surface of the article, and the at least one connector is disposed at a longitudinal end of the article.

7. The article of claim 1, wherein the cover comprises a plurality of closed circuits of heating material that is heatable by penetration with a varying magnetic field;
optionally wherein the article is elongate and the closed circuits are spaced-apart in a longitudinal direction of the article.

8. The article of claim 1, wherein the heating material comprises a plurality of relatively closely-spaced portions of the heating material, and a plurality of less-closely-spaced portions of the heating material.

9. The article of claim 1, wherein the smokable material comprises tobacco and/or one or more humectants.

10. The article of claim 1, further comprising an annular mass of the smokable material, and a liner having a closed circuit of heating material that is heatable by penetration with a varying magnetic field, wherein the annular mass of the smokable material is disposed around the liner such that the liner defines an inner surface of the article.

11. A method of manufacturing an article for use with an apparatus for heating smokable material to volatilize at least one component of the smokable material, the method comprising:
providing (902) smokable material (20); and
providing (904) a cover (10) around the smokable material such that the cover defines an outer surface of the article, wherein the cover comprises a wrapper (12), wherein the cover comprises a closed circuit of heating material that is heatable by penetration with a varying magnetic field, wherein the closed circuit is disposed such that the closed circuit encircles the smokable material, and wherein providing the cover comprises adhering free ends of the wrapper together using an adhesive (14), the method being **characterized in that** the adhesive comprises heating material that is heatable by penetration with a varying magnetic field.

12. A system, comprising:
apparatus configured to heat smokable material and volatilize at least one component of the smokable material; and
an article for use with the apparatus, wherein the article is as defined in any one of claims 1 to 10.

13. The system of claim 12, wherein the apparatus comprises an interface configured to cooperate with the article, and a magnetic field generator configured to generate a varying magnetic field that penetrates the heating material when the article cooperates with the interface.

## Patentansprüche

1. Artikel zur Verwendung mit einer Vorrichtung zur Erwärmung von rauchbarem Material, um mindestens eine Komponente des rauchbaren Materials zu verdampfen, wobei der Artikel Folgendes umfasst:
rauchbares Material (20); und
eine Deckschicht (10) um das rauchbare Material herum;
wobei die Deckschicht eine Außenfläche des Artikels definiert und eine Banderole (12) umfasst, wobei die Deckschicht einen geschlossenen Kreis aus Heizmaterial umfasst, das durch Eindringen mit einem variierenden Magnetfeld erwärmbar ist, wobei der geschlossene Kreis derart angeordnet ist, dass der geschlossene Kreis das rauchbare Material umschließt, und wobei die Deckschicht einen Klebstoff (14) umfasst, der freie Enden der Banderole aneinander klebt, und **dadurch gekennzeichnet, dass** der Klebstoff Heizmaterial umfasst, das durch Eindringen mit einem variierenden Magnetfeld erwärmbar ist.

2. Artikel nach Anspruch 1, wobei das Heizmaterial in Kontakt mit dem rauchbaren Material ist.

3. Artikel nach Anspruch 1, wobei die Deckschicht Folgendes umfasst:
eine erste Banderole, die das rauchbare Material umschließt, wobei die erste Banderole Heizmaterial umfasst, das durch Eindringen mit einem variierenden Magnetfeld erwärmbar ist, eine zweite Banderole, die die erste Banderole umschließt, und einen Klebstoff, der freie Enden der zweiten Banderole aneinander klebt.

4. Artikel nach Anspruch 1, wobei die Deckschicht eine Materialbahn (13) umfasst, wobei das Heizmaterial auf der Materialbahn angeordnet ist;
wobei optional eine Hauptfläche der Materialbahn nur teilweise durch das Heizmaterial bedeckt ist.

5. Artikel nach Anspruch 1, wobei die Deckschicht eine Materialbahn (13) und mehrere einzelne Abschnitte des Heizmaterials in oder auf der Materialbahn umfasst;
wobei optional die einzelnen Abschnitte parallele Streifen sind.

6. Artikel nach Anspruch 1, wobei die Banderole ein Heizmaterial umfasst, das durch Eindringen mit einem variierenden Magnetfeld erwärmbar ist, und
wobei die Deckschicht mindestens einen Verbinder (16) umfasst, der Heizmaterial umfasst, das durch Eindringen mit einem variierenden Magnetfeld erwärmbar ist, wobei der Verbinder mit der Banderole verbunden ist, wobei der geschlossene Kreis das Heizmaterial der Banderole und das Heizmaterial des mindestens einen Verbinders umfasst;
wobei optional der Artikel länglich und zylindrisch ist, die Banderole eine Umfangsaußenfläche des Artikels bildet und der mindestens eine Verbinder an einem Längsende des Artikels angeordnet ist.

7. Artikel nach Anspruch 1, wobei die Deckschicht eine Vielzahl von geschlossenen Kreisen aus Heizmaterial umfasst, das durch Eindringen mit einem variierenden Magnetfeld erwärmbar ist;
wobei optional der Artikel länglich ist und die geschlossenen Kreise in einer Längsrichtung des Artikels beabstandet sind.

8. Artikel nach Anspruch 1, wobei das Heizmaterial eine Vielzahl von relativ eng beabstandeten Abschnitten des Heizmaterials und eine Vielzahl von weniger eng beabstandeten Abschnitten des Heizmaterials umfasst.

9. Artikel nach Anspruch 1, wobei das rauchbare Material Tabak und/oder ein oder mehrere Feuchthaltemittel umfasst.

10. Artikel nach Anspruch 1, ferner umfassend eine ringförmige Masse des rauchbaren Materials und eine Auskleidung mit einem geschlossenen Kreis aus Heizmaterial, das durch Eindringen mit einem variierenden Magnetfeld erwärmbar ist, wobei die ringförmige Masse des rauchbaren Materials um die Auskleidung herum derart angeordnet ist, dass die Auskleidung eine Innenfläche des Artikels definiert.

11. Verfahren zum Herstellen eines Artikels zur Verwendung mit einer Vorrichtung zum Erwärmen von rauchbarem Material, um mindestens eine Komponente des rauchbaren Materials zu verdampfen, wobei das Verfahren Folgendes umfasst:
Bereitstellen (902) von rauchbarem Material (20); und
Bereitstellen (904) einer Deckschicht (10) um das rauchbare Material herum, sodass die Deckschicht eine Außenfläche des Artikels definiert, wobei die Deckschicht eine Banderole (12) umfasst, wobei die Deckschicht einen geschlossenen Kreis aus Heizmaterial umfasst, das durch Eindringen mit einem variierenden Magnetfeld erwärmbar ist, wobei der geschlossene Kreis derart angeordnet ist, dass der geschlossene Kreis das rauchbare Material umschließt, und wobei das Bereitstellen der Deckschicht Kleben von freien Enden der Banderole aneinander unter Verwendung eines Klebstoffs (14), wobei das Verfahren **dadurch gekennzeichnet ist, dass** der Klebstoff Heizmaterial umfasst, das durch Eindringen mit einem variierenden Magnetfeld erwärmbar ist.

12. System, umfassend:
eine Vorrichtung, die zum Erwärmen von rauchbarem Materials und Verdampfen mindestens einer Komponente des rauchbaren Materials konfiguriert ist; und
einen Artikel zur Verwendung mit der Vorrichtung, wobei der Artikel wie in einem der Ansprüche 1 bis 10 definiert ist.

13. System nach Anspruch 12, wobei die Vorrichtung eine Schnittstelle, die dazu konfiguriert ist, mit dem Artikel zusammenzuwirken, und einen Magnetfelderzeuger umfasst, der dazu konfiguriert ist, ein variierendes Magnetfeld zu erzeugen, das in das Heizmaterial eindringt, wenn der Artikel mit der Schnittstelle zusammenwirkt.

## Revendications

1. Article destiné à être utilisé avec un appareil pour chauffer une substance à fumer pour volatiliser au moins un composant de la substance à fumer, l'article comprenant :
une substance à fumer (20) ; et
un couvercle (10) entourant la substance à fumer ;
dans lequel le couvercle définit une surface extérieure de l'article et comprend une enveloppe (12), dans lequel le couvercle comprend un circuit fermé de matériau chauffant pouvant être chauffé par pénétration avec un champ magnétique variable, dans lequel le circuit fermé est disposé de telle sorte que le circuit fermé entoure la substance à fumer, et dans lequel le couvercle comprend un adhésif (14) collant des extrémités libres de l'enveloppe l'une à l'autre, et **caractérisé en ce que** l'adhésif comprend un matériau chauffant pouvant être chauffé par pénétration avec un champ magnétique variable.

2. Article selon la revendication 1, dans lequel le matériau chauffant est en contact avec la substance à fumer.

3. Article selon la revendication 1, dans lequel le couvercle comprend :
une première enveloppe qui entoure la substance à fumer, la première enveloppe comprenant un matériau chauffant pouvant être chauffé par pénétration avec un champ magnétique variable, une seconde enveloppe qui entoure la première enveloppe, et un adhésif collant des extrémités libres de la seconde enveloppe l'une à l'autre.

4. Article selon la revendication 1, dans lequel le couvercle comprend une feuille de matériau (13), dans lequel le matériau chauffant est disposé sur la feuille de matériau ;
éventuellement dans lequel une surface principale de la feuille de matériau n'est que partiellement recouverte par le matériau chauffant.

5. Article selon la revendication 1, dans lequel le couvercle comprend une feuille de matériau (13) et une pluralité de parties discrètes du matériau chauffant dans ou sur la feuille de matériau ;
éventuellement dans lequel les parties discrètes sont des bandes parallèles.

6. Article selon la revendication 1, dans lequel l'enveloppe comprend un matériau chauffant pouvant être chauffé par pénétration avec un champ magnétique variable, et
dans lequel le couvercle comprend au moins un connecteur (16) comprenant un matériau chauffant pouvant être chauffé par pénétration avec un champ magnétique variable, dans lequel le connecteur est connecté à l'enveloppe, dans lequel le circuit fermé comprend le matériau chauffant de l'enveloppe et le matériau chauffant de l'au moins un connecteur ;
éventuellement dans lequel l'article est allongé et cylindrique, l'enveloppe forme une surface extérieure circonférentielle de l'article, et l'au moins un connecteur est disposé au niveau d'une extrémité longitudinale de l'article.

7. Article selon la revendication 1, dans lequel le couvercle comprend une pluralité de circuits fermés de matériau chauffant pouvant être chauffé par pénétration avec un champ magnétique variable ;
éventuellement dans lequel l'article est allongé et les circuits fermés sont espacés dans une direction longitudinale de l'article.

8. Article selon la revendication 1, dans lequel le matériau chauffant comprend une pluralité de parties relativement rapprochées du matériau chauffant, et une pluralité de parties moins rapprochées du matériau chauffant.

9. Article selon la revendication 1, dans lequel la substance à fumer comprend du tabac et/ou un ou plusieurs agents humectants.

10. Article selon la revendication 1, comprenant en outre une masse annulaire de la substance à fumer et une chemise comportant un circuit fermé de matériau chauffant pouvant être chauffé par pénétration avec un champ magnétique variable, dans lequel la masse annulaire de la substance à fumer est disposée autour de la chemise de telle sorte que la chemise définit une surface intérieure de l'article.

11. Procédé de fabrication d'un article destiné à être utilisé avec un appareil pour chauffer une substance à fumer pour volatiliser au moins un composant de la substance à fumer, le procédé comprenant :
la fourniture (902) d'une substance à fumer (20) ; et
la fourniture (904) d'un couvercle (10) autour de la substance à fumer de telle sorte que le couvercle définit une surface extérieure de l'article, dans lequel le couvercle comprend une enveloppe (12), dans lequel le couvercle comprend un circuit fermé de matériau chauffant pouvant être chauffé par pénétration avec un champ magnétique variable, dans lequel le circuit fermé est disposé de telle sorte que le circuit fermé entoure la substance à fumer, et dans lequel la fourniture du couvercle comprend le fait de coller des extrémités libres de l'enveloppe ensemble à l'aide d'un adhésif (14), le procédé étant **caractérisé en ce que** l'adhésif comprend un matériau chauffant pouvant être chauffé par pénétration avec un champ magnétique variable.

12. Système, comprenant :
un appareil configuré pour chauffer une substance à fumer et volatiliser au moins un composant de la substance à fumer ; et un article destiné à être utilisé avec l'appareil, dans lequel l'article est tel que défini selon l'une quelconque des revendications 1 à 10.

13. Système selon la revendication 12, dans lequel l'appareil comprend une interface configurée pour coopérer avec l'article, et un générateur de champ magnétique configuré pour générer un champ magnétique variable qui pénètre le matériau chauffant lorsque l'article coopère avec l'interface.
